# EUROPEAN PATENT SPECIFICATION

(11) **EP 1 948 177 B1**
(45) Date of publication and mention of the grant of the patent: **24.08.2011**
(21) Application number: 06829937.9
(22) Date of filing: 06.11.2006
(51) Int. Cl.: A61K 31/496, A61P 15/00

(54) **USE OF FLIBANSERIN FOR THE TREATMENT OF PRE-MENOPAUSAL SEXUAL DESIRE DISORDERS**
VERWENDUNG VON FLIBANSERIN ZUR BEHANDLUNG VON PRÄMENOPAUSALEN STÖRUNGEN DER SEXUELLEN LUST
UTILISATION DE LA FLIBANSERINE POUR TRAITER LES TROUBLES PREMENOPAUSIQUES DU DESIR SEXUEL

(30) Priority: 08.11.2005 US 734405 P; 14.07.2006 US 831015 P
(43) Date of publication of application: 30.07.2008
(73) Proprietor: Boehringer Ingelheim International GmbH, 55216 Ingelheim am Rhein (DE); Boehringer Ingelheim Pharma GmbH & Co. KG, 55216 Ingelheim am Rhein (DE)
(72) Inventor: POLLENTIER, Stephane, NL-1871 AW Schoorl (NL); PYKE, Robert, New Fairfield, Connecticut 06812 (US)
(74) Representative: Hammann, Heinz
(86) International application number: PCT/EP2006/068118
(87) International publication number: WO 2007/054476

(56) References cited:
- WO-A-03/097058
- WO-A-2005/102342

## Description

The invention relates to the use of flibanserin for the preparation of a medicament for the treatment of pre-menopausal Sexual Desire Disorders.

### Description of the invention

The compound 1-[2-(4-(3-trifluoromethyl-phenyl)piperazin-1-yl)ethyl]-2,3-dihydro-1H-benzimidazol-2-one (flibanserin) is disclosed in form of its hydrochloride in European Patent Application EP-A-526434 and has the following chemical structure:

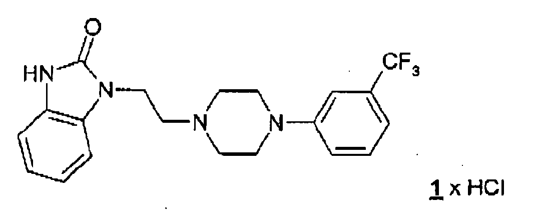

Flibanserin shows affinity for the 5-HT_{1A} and 5-HT₂-receptor. It is therefore a promising therapeutic agent for the treatment of a variety of diseases, for instance depression, schizophrenia, and anxiety.

In WO 03/097058 pharmaceutical formulations comprising Flibanserin are disclosed in WO 05/102342 combinations of Flibanserin with other active ingredients and the use of such combinations for the treatment of sexual disorders is disclosed. The generic term "Sexual Disorders" includes Sexual Desire Disorders, Sexual Arousal Disorders, Orgasmic Disorders, Sexual Pain Disorders, Sexual Dysfunction due to a General Medical Condition, Substance-Induced Sexual Dysfunction, and Sexual Dysfunction not otherwise specified (Diagnostic and Statistical Manual of Mental Disorders, 4th edition, Text Revision. Washington DC, American Psychiatric Association, 2000).

In studies of pre-menopausal female patients suffering from sexual dysfunction it has been found that flibanserin, optionally in form of the free base, the pharmacologically acceptable acid addition salts and/or optionally in form of the hydrates and/or solvates thereof displays sexual desire enhancing properties when applied once daily. Accordingly, the instant invention relates to the use of flibanserin, optionally in form of the free base, the pharmacologically acceptable acid addition salts and/or optionally in form of the hydrates and/or solvates thereof for the preparation of a medicament for the treatment of Sexual Desire Disorders in pre-menopausal women, wherein the medicament is applied one daily consecutively over a period of time.

Within the present invention the terms "treatment of pre-menopausal Hypoactive Sexual Desire Disorder" etc. have the meaning of "treatment of Hypoactive Sexual Desire Disorders in pre-menopausal women" etc.

The beneficial effects of flibanserin can be observed regardless of whether the Sexual Desire Disorder existed lifelong or was acquired, is of the "generalized type" or "situational type"and independent of etiologic origin (organic - both, physically and drug induced-, psychogen (due to psychological factors), a combination of organic - both, physically and drug induced-, and psychogen (due to combined factors), or unknown). The term "lifelong" refers to such Sexual Desire Disorders of the present invention, which have been present since the onset of sexual functioning. The term "acquired" refers to such Sexual Desire Disorders of the present invention which developed only after a period of normal sexual functioning. The "generalized type" refers to such Sexual Disorders of the present invention wherein the disorder is not limited to certain types of stimulation, situations, or partners. The "situational type" applies to such Sexual Disorders of the present invention wherein the disorder is limited to certain types of stimulation, situations, or partners. The subtype due to "psychological factors" applies when psychological factors are judged to have the major role in the onset, severity, exacerbation, or maintenance of the Sexual Disorder, and general medical conditions and substance play no role in the etiology of the Sexual Disorder. Finally the subtype due to "combined factors" applies when 1) psychological factors are judged to have a role in the onset, severity, exacerbation, or maintenance of the Sexual Disorder, and 2) a general medical condition or substance use is also judged to be contributory but is not sufficient to account for a Sexual Disorder (Diagnostic and Statistical Manual of Mental Disorders, 4th edition, Text Revision. Washington DC, American Psychiatric Association, 2000).

Therefore, e.g. the term "lifelong pre-menopausal Hypoactive Sexual Desire Disorder"refers to Hypoactive Sexual Desire Disorder in pre-menopausal women which has been present since the onset of sexual functioning and the term "acquired pre-menopausal Hypoactive Sexual Desire Disorder" refers to Hypoactive Sexual Desire Disorder in pre-menopausal women, which developed after a period of normal sexual functioning.

Accordingly, in a preferred embodiment the invention discloses the use of flibanserin, optionally in form of the free base, the pharmacologically acceptable acid addition salts and/or optionally in form of the hydrates and/or solvates thereof for the preparation of a medicament for the treatment of disorders selected from the group consisting of pre-menopausal Hypoactive Sexual Desire Disorder (HSDD), pre-menopausal Sexual Aversion Disorder, pre-menopausal loss of sexual desire, pre-menopausal lack of sexual desire, pre-menopausal decreased sexual desire, pre-menopausal inhibited sexual desire, pre-menopausal loss of libido, pre-menopausal libido disturbance, and pre-menopausal frigidity.

Disclosed as particular preferred according to the invention is the use of flibanserin, optionally in form of the free base, the pharmacologically acceptable acid addition salts and/or optionally in form of the hydrates and/or solvates thereof for the preparation of a medicament for the treatment of disorders selected from the group consiting of pre-menopausal Hypoactive Sexual Desire Disorder, pre-menopausal Sexual Aversion Disorder, pre-menopausal loss of sexual desire, pre-menopausal lack of sexual desire, pre-menopausal decreased sexual desire, and pre-menopausal inhibited sexual desire.

In a particularily preferred embodiment the invention discloses the use of flibanserin, optionally in form of the free base, the pharmacologically acceptable acid addition salts and/or optionally in form of the hydrates and/or solvates thereof for the preparation of a medicament for the treatment of disorders selected from the group of pre-menopausal Hypoactive Sexual Desire Disorder, pre-menopausal loss of sexual desire, pre-menopausal decreased sexual desire, and pre-menopausal inhibited sexual desire.

In a further preferred embodiment the invention discloses the use of flibanserin, optionally in form of the free base, the pharmacologically acceptable acid addition salts and/or optionally in form of the hydrates and/or solvates thereof for the preparation of a medicament for the treatment of disorders selected from the group consisting of lifelong pre-menopausal Hypoactive Sexual Desire Disorder, lifelong pre-menopausal Sexual Aversion Disorder, lifelong pre-menopausal loss of sexual desire, lifelong pre-menopausal lack of sexual desire, lifelong pre-menopausal decreased sexual desire, lifelong pre-menopausal inhibited sexual desire, lifelong pre-menopausal loss of libido, lifelong pre-menopausal libido disturbance, and lifelong pre-menopausal frigidity.

Disclosed as particular preferred according to the invention is the use of flibanserin, optionally in form of the free base, the pharmacologically acceptable acid addition salts and/or optionally in form of the hydrates and/or solvates thereof for the preparation of a medicament for the treatment of disorders selected from the group consiting of lifelong pre-menopausal Hypoactive Sexual Desire Disorder, lifelong pre-menopausal Sexual Aversion Disorder, lifelong pre-menopausal loss of sexual desire, lifelong pre-menopausal lack of sexual desire, lifelong pre-menopausal decreased sexual desire, and lifelong pre-menopausal inhibited sexual desire.

In a particularily preferred embodiment the invention discloses the use of flibanserin, optionally in form of the free base, the pharmacologically acceptable acid addition salts and/or optionally in form of the hydrates and/or solvates thereof for the preparation of a medicament for the treatment of disorders selected from the group of lifelong pre-menopausal Hypoactive Sexual Desire Disorder, lifelong pre-menopausal loss of sexual desire, lifelong pre-menopausal decreased sexual desire, and lifelong pre-menopausal inhibited sexual desire.

In a further preferred embodiment the invention discloses the use of flibanserin, optionally in form of the free base, the pharmacologically acceptable acid addition salts and/or optionally in form of the hydrates and/or solvates thereof for the preparation of a medicament for the treatment of disorders selected from the group consisting of acquired pre-menopausal Hypoactive Sexual Desire Disorder, acquired pre-menopausal Sexual Aversion Disorder, acquired pre-menopausal loss of sexual desire, acquired pre-menopausal lack of sexual desire, acquired pre-menopausal decreased sexual desire, acquired pre-menopausal inhibited sexual desire, acquired pre-menopausal loss of libido, acquired pre-menopausal libido disturbance, and acquired pre-menopausal frigidity.

Furthermore disclosed as preferred according to the invention is the use of flibanserin, optionally in form of the free base, the pharmacologically acceptable acid addition salts and/or optionally in form of the hydrates and/or solvates thereof for the preparation of a medicament for the treatment of disorders selected from the group consiting of acquired pre-menopausal Hypoactive Sexual Desire Disorder, acquired pre-menopausal Sexual Aversion Disorder, acquired pre-menopausal loss of sexual desire, acquired pre-menopausal lack of sexual desire, acquired pre-menopausal decreased sexual desire, acquired pre-menopausal inhibited sexual desire.

In a particularily disclosed as preferred embodiment the invention relates to the use of flibanserin, optionally in form of the free base, the pharmacologically acceptable acid addition salts and/or optionally in form of the hydrates and/or solvates thereof for the preparation of a medicament for the treatment of disorders selected from the group of acquired pre-menopausal Hypoactive Sexual Desire Disorder, acquired pre-menopausal loss of sexual desire, acquired pre-menopausal decreased sexual desire and acquired pre-menopausal inhibited sexual desire.

Furthermore the present invention discloses the generalized or situational subtype of any of the above mentioned conditions and/or to such which are due to organic factors, psychological factors or due to combined factors.

Flibanserin can optionally used in form of the free base, in form of its pharmaceutically acceptable acid addition salts and/or optionally in form of the hydrates and/or solvates thereof. Suitable acid addition salts include for example those of the acids selected from, succinic acid, hydrobromic acid, acetic acid, fumaric acid, maleic acid, methanesulphonic acid, lactic acid, phosphoric acid, hydrochloric acid, sulphuric acid, tartaric acid and citric acid. Mixtures of the abovementioned acid addition salts may also be used. From the aforementioned acid addition salts the hydrochloride and the hydrobromide, particularily the hydrochloride, are preferred. If flibanserin is used in form of the free base, it is preferably used in form of flibanserin polymorph A as disclosed in WHO 03/014079.

Flibanserin, optionally used in form of the free base, the pharmacologically acceptable acid addition salts and/or optionally in form of the hydrates and/or solvates thereof, may be incorporated into the conventional pharmaceutical preparation in solid, liquid or spray form. The composition may, for example, be presented in a form suitable for oral, rectal, parenteral administration or for nasal inhalation: preferred forms includes for example, capsules, tablets, coated tablets, ampoules, suppositories and nasal spray.

The active ingredient may be incorporated in excipients or carriers conventionally used in pharmaceutical compositions such as, for example, talc, arabic gum, lactose, gelatine, magnesium stearate, corn starch, acqueous or non acqueous vehicles, polyvynil pyrrolidone, semisynthetic glicerides of fatty acids, benzalconium chloride, sodium phosphate , EDTA, polysorbate 80. The compositions are advantageously formulated in dosage units, each dosage unit being adapted to supply a single dose of the active ingredient. The dosis range applicable per day is between 0.1 to 400, preferably between 1.0 to 300, more preferably between 2 to 200 mg.

Each dosage unit may conveniently contain from 0,01 mg to 100 mg, preferably from 0,1 to 50 mg.

The dosage units are administered to the patient 1, 2, 3, or 4 times daily. It is preferred that the compounds of the invention be administered either three or fewer times, more preferably once or twice daily consecutively over a period of time.

Preferably, the dose is administered to a patient in the morning and the evening, more preferably once in the morning (25 or 50 mg of flibanserin) and once in the evening (25 or 50 mg of flibanserin), most preferably once in the evening only (50 or 100 mg of flibanserin) consecutively over a period of time.

As a result side-effects such as sedation are of lesser significance.

Suitable tablets may be obtained, for example, by mixing the active substance(s) with known excipients, for example inert diluents such as calcium carbonate, calcium phosphate or lactose, disintegrants such as corn starch or alginic acid, binders such as starch or gelatine, lubricants such as magnesium stearate or talc and/or agents for delaying release, such as carboxymethyl cellulose, cellulose acetate phthalate, or polyvinyl acetate. The tablets may also comprise several layers.

Coated tablets may be prepared accordingly by coating cores produced analogously to the tablets with substances normally used for tablet coatings, for example collidone or shellac, gum arabic, talc, titanium dioxide or sugar. To achieve delayed release or prevent incompatibilities the core may also consist of a number of layers. Similarly the tablet coating may consist of a number or layers to achieve delayed release, possibly using the excipients mentioned above for the tablets.

Syrups or elixirs containing the active substances or combinations thereof according to the invention may additionally contain a sweetener such as saccharine, cyclamate, glycerol or sugar and a flavour enhancer, e.g of, a flavouring such as vanilline or orange extract. They may also contain suspension adjuvants or thickeners such as sodium carboxymethyl cellulose, wetting agents such as, for example, condensation products of fatty alcohols with ethylene oxide, or preservatives such as p-hydroxybenzoates.

Solutions for injection are prepared in the usual way, e.g of, with the addition of preservatives such as p-hydroxybenzoates, or stabilisers such as alkali metal salts of ethylenediamine tetraacetic acid, and transferred into injection vials or ampoules. Capsules containing one or more active substances or combinations of active substances may for example be prepared by mixing the active substances with inert carriers such as lactose or sorbitol and packing them into gelatine capsules.

Suitable suppositories may be made for example by mixing with carriers provided for this purpose, such as neutral fats or polyethyleneglycol or the derivatives thereof.

The Examples which follow illustrate the present invention without restricting its scope:

### Examples of pharmaceutical formulations

| A) | Tablets | per tablet |
|---|---|---|
| | flibanserin | 100 mg |
| | lactose | 240 mg |
| | corn starch | 340 mg |
| | polyvinylpyrrolidone | 45 mg |
| | magnesium stearate | 15 mg |
| | | 740 mg |

The finely ground active substance, lactose and some of the corn starch are mixed together. The mixture is screened, then moistened with a solution of polyvinylpyrrolidone in water, kneaded, wet-granulated and dried. The granules, the remaining corn starch and the magnesium stearate are screened and mixed together. The mixture is compressed to produce tablets of suitable shape and size.

| B) | Tablets | per tablet |
|---|---|---|
| | flibanserin | 80 mg |
| | corn starch | 190 mg |
| | lactose | 55 mg |
| | microcrystalline cellulose | 35 mg |
| | polyvinylpyrrolidone | 15 mg |
| | sodium-carboxymethyl starch | 23 mg |
| | magnesium stearate | 2 mg |
| | | 400 mg |

The finely ground active substance, some of the corn starch, lactose, microcrystalline cellulose and polyvinylpyrrolidone are mixed together, the mixture is screened and worked with the remaining corn starch and water to form a granulate which is dried and screened. The sodium-carboxymethyl starch and the magnesium stearate are added and mixed in and the mixture is compressed to form tablets of a suitable size.

| C) | Coated tablets | *per coated tablet* |
|---|---|---|
| | flibanserin | 5 mg |
| | corn starch | 41.5 mg |
| | lactose | 30 mg |
| | polyvinylpyrrolidone | 3 mg |
| | magnesium stearate | 0.5 mg |
| | | 80 mg |

The active substance, corn starch, lactose and polyvinylpyrrolidone are thoroughly mixed and moistened with water. The moist mass is pushed through a screen with a 1 mm mesh size, dried at about 45°C and the granules are then passed through the same screen. After the magnesium stearate has been mixed in, convex tablet cores with a diameter of 6 mm are compressed in a tablet-making machine. The tablet cores thus produced are coated in known manner with a covering consisting essentially of sugar and talc. The finished coated tablets are polished with wax.

| D) | Capsules | per capsule |
|---|---|---|
| | flibanserin | 1 50 mg |
| | Corn starch . | 268.5 mg |
| | Magnesium stearate | 1.5 mg |
| | | 420 mg |

The substance and corn starch are mixed and moistened with water. The moist mass is screened and dried. The dry granules are screened and mixed with magnesium stearate. The finished mixture is packed into size 1 hard gelatine capsules.

| E) | Ampoule solution | |
|---|---|---|
| | flibanserin | 50 mg |
| | sodium chloride | 50 mg |
| | water for inj. | 5 ml |

The active substance is dissolved in water at its own pH or optionally at pH 5.5 to 6.5 and sodium chloride is added to make it isotonic. The solution obtained is filtered free from pyrogens and the filtrate is transferred under aseptic conditions into ampoules which are then sterilised and sealed by fusion.

| F) | Suppositories | |
|---|---|---|
| | flibanserin | 50 mg |
| | solid fat | 1650 mg |
| | | 1700 mg |

The hard fat is melted. At 40°C the ground active substance is homogeneously dispersed. It is cooled to 38°C and poured into slightly chilled suppository moulds.

### Results of Clinical Trials

In the following, experimental data of a clinical trial proving the effect of flibanserin in the treatment of Sexual Desire Disorders in pre-menopausal women are presented.

This trial was designed as a prospective, multi-center, twelve-week, randomized, double-blind, placebo-controlled, proof of concept, parallel-group trial comparing the effects of flibanserin (maximum total daily dose: 100 mg b.i.d.) to placebo in pre-menopausal female patients with HSDD. Seventy-five patients were to be randomized to each treatment group.

This proof of concept trial was designed to assess whether twelve weeks of flibanserin treatment produced a clinically meaningful therapeutic response in healthy female patients with HSDD (as determined by DSM-IV criteria). Efficacy for flibanserin was assessed versus a parallel placebo group.

After a Screening period (no treatment) of approximately twenty-eight days, eligible patients were randomized into the twelve week, double-blind portion of the trial during which they were to take study medication in the morning and in the evening about 12 hours apart.

| **Trial Periods** | **Screening** | **Baseline** | **Treatment** | | **Final Visit** |
|---|---|---|---|---|---|
| Visit | 1 | 2 | 3 | 4 | 5 |
| Day | -28 +/- 3 | 0 | 28 +/- 3 | 56 +/- 3 | 84 +/- 3 |
| Week | -4 | 0 | 4 | 8 | 12 |

Patients must have been pre-menopausal females who were 18 to 45 years of age with the primary diagnosis of HSDD, acquired type, according to DSM-IV criteria. The current episode must have been at least 24 weeks in duration by the Baseline Visit.

The baseline severity criterion was from the Arizona Sexual Experiences Scale, requiring a score of 5 or 6 (very weak or no sex drive) on the sex drive item. (McGahuey CA. et al., Psychiatric Annals 1999; 29(1): 39-45; McGahuey CA. et al., J. Sex Marital Therapy 2000; 26: 25-44).

The assignment of doses was a simple random assignment with the possibility of a one-time up-titration at Week 8. The starting dosage was to be one tablet in the morning and one tablet in the evening.

Patients were instructed to take the blinded study medication as close to every twelve hours as possible. It was recommended that doses not be taken less than ten hours apart. If a dose was missed, the next regular dose was to be taken as scheduled. No double doses were to be taken. Patients were advised that each dose of study medication was to be taken with 150 millimeter (five ounces) of water.

If the patient was not showing meaningful improvement at Day 56 (Week 8) in the investigator's opinion and had no severe or intolerable adverse events, the number of tablets per day was to be doubled from one tablet each morning and evening, increasing the dose of flibanserin from 50 mg b.i.d. to 100 mg b.i.d, or doubling the number of placebo tablets from two per day to four per day for patients in the placebo group.

As one efficacy variable to prove efficacy of flibanserin in the treatment of HSDD in pre-menopausal women, the Interactive Voice Response-Female Sexual Behavior Questionnaire (IVR-FSBQ) was designed as a simple self-administered questionnaire to be completed using a telephone to measure sexual desire-related feelings and events. To facilitate compliance with its use, the FSBQ was to be used in this trial on a weekly basis via an IVR System developed and administered by Healthcare Technology Systems, Inc.

The IVR-FSBQ (as far as related to desire) is shown below.
1. How often did you engage in sexual thoughts such as thinking about having sex or sexual fantasies, this past week?
   *If not at all in the past week - Press 0*
   *If on one day in the past week - Press 1*
   *If on two days in the past week - Press 2*
   *If on three days or more but not every day in the past week - Press 3*
   *If everyday this past week - Press 4*
   *If more than once per day in the past week - Press 5*

Analyses of endpoints were performed on the FAS (Full Analysis Dataset). The LOCF method (Last Observation carried forward) of data estimation was used unless otherwise specified.

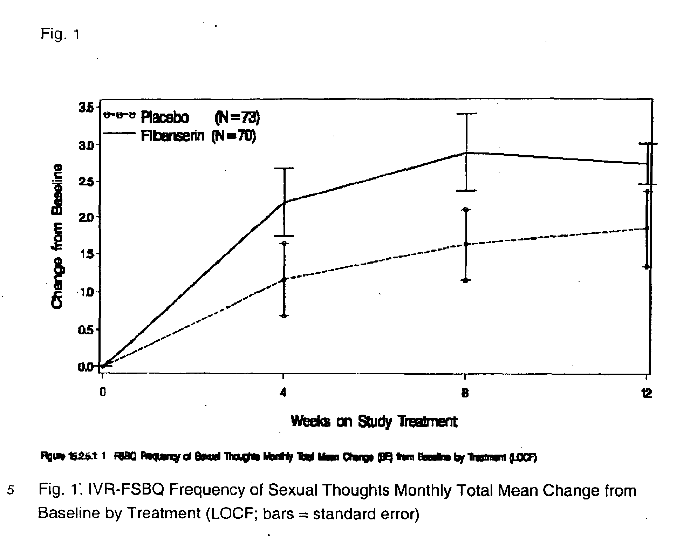

To meet the DSM-IV criteria for Hypoactive Sexual Desire Disorder, the severity requirement on lack of desire is "persistently or recurrently deficient (or absent) sexual fantasies and desire for sexual activity." Thus, IVR-FSBQ question 1, ""How often did you engage in sexual thoughts such as thinking about having sex or sexual fantasies, this past week?" is of the essence in proving whether flibanserin treats Sexual Desire Disorders. One major result of this clinical trial was the difference on this question, namely in the monthly mean change from baseline, between patients treated with flibanserin and placebo. A graph for IVR-FSBQ Monthly Mean Change from Baseline scores for Frequency of Sexual Thoughts is displayed in Fig. 1., which clearly demonstrates the efficacy of flibanserin in the treatment of Sexual Desire Disorders in pre-menopausal women.

## Claims

1. Use of flibanserin, optionally in form of the free base, the pharmacologically acceptable acid addition salts and/or optionally in form of the hydrates and/or solvates thereof for the preparation of a medicament for the treatment of pre-menopausal Sexual Desire Disorders in women, wherein the medicament is applied once daily consecutively over a period of time.

2. Use according to claim 1, **characterized in that** the pre-menopausal Sexual Desire Disorder is selected from the group consisting of pre-menopausal Hypoactive Sexual Desire Disorder, pre-menopausal Sexual Aversion Disorder, pre-menopausal loss of sexual desire, pre-menopausal lack of sexual desire, pre-menopausal decreased sexual desire, pre-menopausal inhibited sexual desire, pre-menopausal loss of libido, pre-menopausal libido disturbance, and pre-menopausal frigidity.

3. Use according to claim 1, **characterized in that** the pre-menopausal Sexual Desire Disorder is pre-menopausal Hypoactive Sexual Desire Disorder.

4. Use according to claim 1, 2 or 3 **characterized in that** the pre-menopausal Sexual Desire Disorders are of lifelong type.

5. Use according to claim 1, 2 or 3 **characterized in that** the pre-menopausal Sexual Desire Disorders are of acquired type.

6. Use according to one or more of the preceding claims, **characterized in that** the pre-menopusal Sexual Desire Disorders are of the generalized subtype.

7. Use according to one or more of the preceding claims, **characterized in that** the pre-menopusal Sexual Desire Disorders are of the situational subtype.

8. Use according to one or more of the preceding claims, **characterized in that** the pre-menopusal Sexual Desire Disorders are due to psychological factors.

9. Use according to one or more of the preceding claims, **characterized in that** the pre-menopusal Sexual Desire Disorders are due to organic factors.

10. Use according to one or more of the preceding claims, **characterized in that** the pre-menopusal Sexual Desire Disorders are due to combined factors.

11. Use according to one or more of the preceding claims **characterized in that** flibanserin is applied in form of a pharmaceutically acceptable acid addition salt selected from the salts formed by the acids selected from, succinic acid, hydrobromic acid, acetic acid, fumaric acid, maleic acid, methanesulphonic acid, lactic acid, phosphoric acid, hydrochloric acid, sulphuric acid, tartaric acid, citric acid, and mixtures thereof.

12. Use according to one or more of the preceding claims, **characterized in that** flibanserin is applied in form of its free base.

13. Use according to claim 12, **characterized in that** flibanserin is applied in form of a polymorph A of the free base, having a melting point of about 161 °C as measured using DSC.

14. Use according to one or more of the preceding claims, **characterized in that** flibanserin is applied once in the evening only (50 or 100 mg of flibanserin) consecutively over a period of time.

## Patentansprüche

1. Verwendung von Flibanserin, gegebenenfalls in Form der freien Base, den pharmakologisch akzeptablen Säureadditionssalzen und/oder gegebenenfalls in Form der Hydrate und/oder Solvate davon zur Herstellung eines Arzneimittels zur Behandlung von prämenopausalen sexuellen Begehrensstörungen bei Frauen, wobei das Arzneimittel einmal täglich fortlaufend über eine Zeitspanne angewandt wird.

2. Verwendung nach Anspruch 1, **dadurch gekennzeichnet, dass** die prämenopausale sexuelle Begehrensstörung ausgewählt wird aus der Gruppe, bestehend aus prämenopausaler hypoaktiver sexueller Begehrensstörung, prämenopausaler sexueller Aversionsstörung bzw. Abneigungsstörung, prämenopausalem Verlust des sexuellen Verlangens, prämenopausalem Mangel an sexuellem Verlangen, prämenopausal verringertem sexuellen Verlangen, prämenopausal unterdrücktem sexuellen Verlangen, prämenopausalem Verlust der Libido, prämenopausaler Libidostörung und prämenopausaler Frigidität.

3. Verwendung nach Anspruch 1, **dadurch gekennzeichnet, dass** die prämenopausale sexuelle Begehrensstörung prämenopausale hypoaktive sexuelle Begehrensstörung darstellt.

4. Verwendung nach Anspruch 1, 2 oder 3, **dadurch gekennzeichnet, dass** die prämenopausalen sexuellen Begehrensstörungen lebenslang vorliegen.

5. Verwendung nach Anspruch 1, 2 oder 3, **dadurch gekennzeichnet, dass** die prämenopausalen sexuellen Begehrensstörungen erworben wurden.

6. Verwendung nach einem oder mehreren der vorhergehenden Ansprüche, **dadurch gekennzeichnet, dass** die prämenopausalen sexuellen Begehrensstörungen den verallgemeinerten Subtyp darstellen.

7. Verwendung nach einem oder mehreren der vorhergehenden Ansprüche, **dadurch gekennzeichnet, dass** die prämenopausalen sexuellen Begehrensstörungen den situationsbedingten Subtyp darstellen.

8. Verwendung nach einem oder mehreren der vorhergehenden Ansprüche, **dadurch gekennzeichnet, dass** die prämenopausalen sexuellen Begehrensstörungen auf psychologischen Faktoren beruhen.

9. Verwendung nach einem oder mehreren der vorhergehenden Ansprüche, **dadurch gekennzeichnet, dass** die prämenopausalen sexuellen Begehrensstörungen auf organischen Faktoren beruhen.

10. Verwendung nach einem oder mehreren der vorhergehenden Ansprüche, **dadurch gekennzeichnet, dass** die prämenopausalen sexuellen Begehrensstörungen auf kombinierten Faktoren beruhen.

11. Verwendung nach einem oder mehreren der vorhergehenden Ansprüche, **dadurch gekennzeichnet, dass** Flibanserin in Form eines pharmazeutisch akzeptablen Säureadditionssalz angewandt wird, ausgewählt aus den Salzen, gebildet mit den Säuren, ausgewählt aus Bernsteinsäure, Bromwasserstoffsäure, Essigsäure, Fumarsäure, Maleinsäure, Methansulfonsäure, Milchsäure, Phosphorsäure, Salzsäure, Schwefelsäure, Weinsäure, Zitronensäure und Mischungen hiervon.

12. Verwendung nach einem oder mehreren der vorhergehenden Ansprüche, **dadurch gekennzeichnet, dass** Flibanserin in Form der freien Base angewandt wird.

13. Verwendung nach Anspruch 12, **dadurch gekennzeichnet, dass** Flibanserin in Form eines Polymorphs A der freien Base mit einem Schmelzpunkt von etwa 161°C, wie gemessen unter Verwendung von DSC, angewandt wird.

14. Verwendung nach einem oder mehreren der vorhergehenden Ansprüche, **dadurch gekennzeichnet, dass** Flibanserin nur einmal Abends (50 oder 100 mg Flibanserin) fortlaufend für eine Zeitspanne angewandt wird.

## Revendications

1. Utilisation de la flibansérine, éventuellement sous la forme de la base libre, des sels d'addition d'acide pharmacologiquement acceptables et/ou éventuellement sous la forme d'hydrates et/ou de solvate de celle-ci pour la préparation d'un médicament destiné au traitement de troubles préménopausiques du désir sexuel chez les femmes, où le médicament est appliqué une fois par jour de manière consécutive sur une période de temps.

2. Utilisation selon la revendication 1, **caractérisée en ce que** le trouble préménopausique du désir sexuel est choisi dans le groupe comprenant un trouble préménopausique hypoactif du désir sexuel, un trouble préménopausique d'aversion sexuelle, une perte préménopausique du désir sexuel, un manque préménopausique de désir sexuel, une diminution préménopausique du désir sexuel, une inhibition préménopausique du désir sexuel, une perte préménopausique de la libido, une perturbation préménopausique de la libido et une frigidité préménopausique.

3. Utilisation selon la revendication 1, **caractérisée en ce que** le trouble préménopausique du désir sexuel est un trouble préménopausique hypoactif du désir sexuel.

4. Utilisation selon la revendication 1, 2 ou 3, **caractérisée en ce que** les troubles préménopausiques du désir sexuel sont de type la vie durant.

5. Utilisation selon la revendication 1, 2 ou 3, **caractérisée en ce que** les troubles préménopausiques du désir sexuel sont de type acquis.

6. Utilisation selon une ou plusieurs des revendications précédentes, **caractérisée en ce que** les troubles préménopausiques du désir sexuel sont du sous-type généralisé.

7. Utilisation selon une ou plusieurs des revendications précédentes, **caractérisée en ce que** les troubles préménopausiques du désir sexuel sont du sous-type situationnel.

8. Utilisation selon une ou plusieurs des revendications précédentes, **caractérisée en ce que** les troubles préménopausiques du désir sexuel sont dus à des facteurs psychologiques.

9. Utilisation selon une ou plusieurs des revendications précédentes, **caractérisée en ce que** les troubles préménopausiques du désir sexuel sont dus à des facteurs organiques.

10. Utilisation selon une ou plusieurs des revendications précédentes, **caractérisée en ce que** les troubles préménopausiques du désir sexuel sont dus à des facteurs combinés.

11. Utilisation selon une ou plusieurs des revendications précédentes, **caractérisée en ce que** la flibansérine est appliquée sous la forme d'un sel d'addition d'acide pharmaceutiquement acceptable choisi parmi les sels formés par les acides choisis parmi l'acide succinique, l'acide bromhydrique, l'acide acétique, l'acide fumarique, l'acide maléique, l'acide méthane sulfonique, l'acide lactique, l'acide phosphorique, l'acide chlorhydrique, l'acide sulfurique, l'acide tartrique, l'acide citrique et des mélanges de ceux-ci.

12. Utilisation selon une ou plusieurs des revendications précédentes, **caractérisée en ce que** la flibansérine est appliquée sous la forme de sa base libre.

13. Utilisation selon la revendication 12, **caractérisée en ce que** la flibansérine est appliquée sous la forme d'un polymorphe A de la base libre, présentant un point de fusion d'environ 161 °C mesuré à l'aide d'une DSC.

14. Utilisation selon une ou plusieurs des revendications précédentes, **caractérisée en ce que** la flibansérine est appliquée une fois dans la soirée uniquement (50 ou 100 mg de flibansérine) de manière consécutive sur une période de temps.
